# EUROPEAN PATENT APPLICATION

(11) **EP 3 000 387 A1**
(43) Date of publication of application: **30.03.2016**
(21) Application number: 15182837.3
(22) Date of filing: 28.08.2015
(51) Int. Cl.: A61B 5/00, A61B 5/117, G06F 19/00

(54) **MEDICAL SYSTEM**

(30) Priority: 24.09.2014 JP 2014193978
(71) Applicant: Nihon Kohden Corporation, Shinjuku-ku Tokyo (JP)
(72) Inventor: SUGIURA, Hiroyasu, Shinjuku-ku, Tokyo (JP)
(74) Representative: Grünecker Patent- und Rechtsanwälte PartG mbB

(57) **Abstract**

A medical system includes: a transmitter (200) which is configured to acquire biological information of a patient, and which is configured to transmit the biological information through an inherent channel; a setting terminal (300) which is configured to recognize identification information for identifying a patient, which is configured to acquire the channel from the transmitter, and which is configured to transmit association information in which the identification information is associated with the channel; and a receiver (400) which, by using the association information transmitted from the setting terminal, is configured to register the identification information and the channel in association with each other.

## Description

### BACKGROUND

The present invention relates to a medical system.

There is a technique for detecting and displaying biological information of a patient such as the heart rate and the respiratory rate (for example, see JP-A-2002-191565). There is also a medical system in which a plurality of transmitters for detecting and wirelessly transmitting biological information of patients, and a receiver for receiving and displaying the biological information are used so that the detection functions and the display function are dispersed. The plurality of transmitters are allocated respectively with different inherent channels so that mutual interference does not occur. For example, the channels correspond to different frequencies, respectively. The receiver receives the sets of biological information from the plural transmitters, and can display simultaneously or selectively the sets of biological information.

The receiver must display the sets of biological information transmitted from the plural transmitters, while clearly indicating the patients to whom the sets of biological information belong, respectively. In the receiver, for example, the channel of each of transmitters, and patient identification information such as the ID and name of the patient to whom the transmitter is attached are registered in association with each other, so that the biological information and the patient identification information can be displayed in association with each other.

In the above described configuration, however, the work of inputting and associating the channel and patient identification information with each other is manually performed by the medical staff member. Therefore, there is a possibility that patient identification information of a certain patient may be associated with the channel of the transmitter for another patient, or with an erroneous channel. In such a situation, it is impossible to correctly monitor patient identification information of a patient. This is problematic. In a medical site where speed and correctness are required, it is desired to easily and correctly associate patient identification information with the channel of a transmitter.

### SUMMARY

The invention may provide a medical system in which patient identification information and the channel of a transmitter can be associated with each other easily and correctly.

The medical system may comprise: a transmitter which is configured to acquire biological information of a patient, and which is configured to transmit the biological information through an inherent channel; a setting terminal which is configured to recognize identification information for identifying a patient, which is configured to acquire the channel from the transmitter, and which is configured to transmit association information in which the identification information is associated with the channel; and a receiver which, by using the association information transmitted from the setting terminal, is configured to register the identification information and the channel in association with each other.

### BRIEF DESCRIPTION OF THE DRAWINGS

Fig. 1 is a diagram schematically illustrating the configuration of a medical system of a first embodiment.
Fig. 2 is a block diagram illustrating the functional configuration of the medical system of the first embodiment.
Fig. 3 is a sequence diagram illustrating the process procedure in the medical system of the first embodiment.
Fig. 4 illustrates the manner of processing in the medical system of the first embodiment.
Fig. 5 is a sequence diagram illustrating the process procedure in a medical system of a second embodiment.
Fig. 6 illustrates the manner of processing in the medical system of the second embodiment.
Fig. 7 is a flowchart illustrating the procedure of an admit completion inquiring process which is performed in an admit instruction receiving process shown in step S201 in Fig. 5.

### DETAILED DESCRIPTION OF EXEMPLARY EMBODIMENTS

Hereinafter, embodiments of the invention will be described with reference to the accompanying drawings. The identical components in the figures are denoted by the same reference numerals, and duplicated description is omitted. In the drawings, the dimension ratios are exaggerated for the sake of convenience in description, and may be sometimes different from the actual ratios.

### (First embodiment)

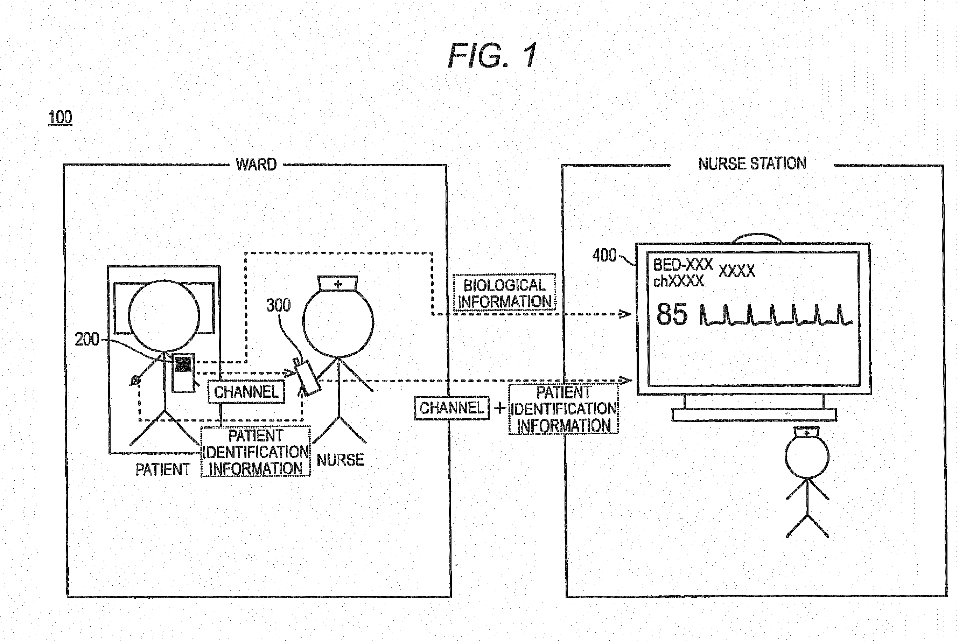

Fig. 1 is a diagram schematically illustrating the configuration of a medical system of a first embodiment.

The medical system of the first embodiment is disposed in medical facility, for example, a hospital.

As shown in Fig. 1, the medical system 100 may have a transmitter 200, a setting terminal 300, and a receiver 400.

The transmitter 200 is attached to, for example, a patient in a ward, acquires biological information of the patient, and wirelessly transmits the biological information to the receiver 400.

The setting terminal 300 is carried by, for example, a nurse who is a medical staff member, and acquires patient identification information for identifying the patient, and the channel of the transmitter 200 attached to the patient. The setting terminal 300 recognizes a patient tag or the like attached to the patient, to acquire the patient identification information for identifying the patient. The setting terminal 300 automatically acquires the channel of the transmitter 200 which is closest to the terminal. The setting terminal 300 associates the acquired patient identification information with the acquired channel, and transmits the associated patient identification information and channel (hereinafter, referred to as association information) to the receiver 400.

The receiver 400 is installed in, for example, a nurse station, receives the association information from the setting terminal 300, and registers the received association information. The receiver 400 further receives biological information from the transmitter 200, and displays the received biological information and the like on a display or the like.

The configurations of the components will be described in detail.

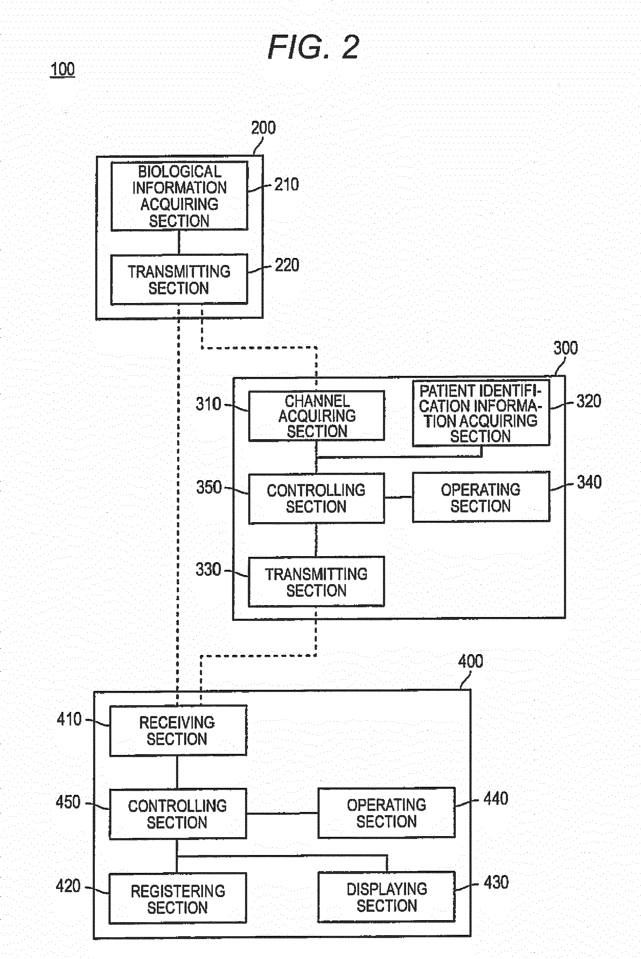

Fig. 2 is a block diagram illustrating the functional configuration of the medical system of the first embodiment.

### (Transmitter 200)

As shown in Fig. 2, the transmitter 200 may include a biological information acquiring section 210 and a transmitting section 220.

The biological information acquiring section 210 acquires biological information of the patient. For example, the biological information acquiring section 210 is configured by electrodes for measuring an electrocardiogram and a probe for measuring the blood oxygen saturation. The biological information acquired by the biological information acquiring section 210 includes an electrocardiogram signal, the blood oxygen saturation, the blood pressure, the heart rate, the body temperature, and the like. The biological information acquiring section 210 sends the acquired biological information to the transmitting section 220.

The transmitting section 220 wirelessly transmits the biological information acquired by the biological information acquiring section 210, to the receiver 400. Usually, one transmitter 200 is attached to one patient. When there are a plurality of patients, therefore, a plurality of transmitters 200 exist. Different channels are allocated to the plurality of transmitters 200, respectively. The transmitting sections 220 of the transmitters 200 transmit biological information through different respective channels.

### (Setting terminal 300)

As shown in Fig. 2, the setting terminal 300 may include a channel acquiring section 310, a patient identification information acquiring section 320, a transmitting section 330, an operating section 340, and a controlling section 350.

The channel acquiring section 310 acquires from the transmitter 200 closest to the terminal 300, the channel which is used in the transmitter 200. When a predetermined operation such as an operation of pressing a button of the operating section 340 is received, for example, the channel acquiring section 310 receives wireless signals while sequentially switching the channel to be received. The channel acquiring section 310 acquires the channel in which the received wireless signal has the highest intensity, as the channel of the transmitter 200 closest to the terminal. The larger the distance from the signal generation source (transmitter 200) is, the more the intensity of a wireless signal attenuates. If the signal intensities in the signal generation sources of the transmitters 200 are identical to one another, therefore, the signal which is transmitted by using the channel of the transmitter 200 closest to the setting terminal 300 has the maximum intensity. Consequently, the setting terminal 300 can automatically acquire the channel of the transmitter 200 closest to the terminal, by acquiring the channel in which the intensity of the wireless signal is highest.

For example, the patient identification information acquiring section 320 is configured by a camera, a bar code reader, an RFID tag reader, or the like. When the section 320 recognizes a patient tag or the like attached to the patient, the section 320 acquires the patient identification information for identifying the patient. The patient identification information contains information for identifying the patient, such as the name, ID, and head shot of the patient. For example, the patient identification information acquiring section 320 reads a bar code which is printed on the patient tag, to acquire the patient ID which is coded with a bar code, as patient identification information. Alternatively, the patient identification information acquiring section 320 may take an image of the name of the patient which is printed on the patient tag, with a camera, and apply image processing such as character recognition to the taken image, thereby acquiring the name of the patient as patient identification information. The patient identification information acquiring section 320 may take an image of the face of the patient, and acquire the taken face image as patient identification information. The patient identification information acquiring section 320 may apply image processing such as facial recognition to the taken face image, and perform matching with a database which is previously stored in a storing section, thereby acquiring the name or the like of the patient from the face image.

The transmitting section 330 associates the channel which is acquired by the channel acquiring section 310, with the patient identification information which is acquired by the patient identification information acquiring section 320, and transmits them as the association information to the receiver 400.

The operating section 340 is configured by, for example, buttons which are disposed as hardware on the setting terminal 300, and receives various inputs and the like from the user. Alternatively, the operating section 340 may be configured by operation keys which are realized as software by a touch panel in a displaying section (not shown) of the setting terminal 300.

The controlling section 350 is configured by, for example, a CPU, controls the above-described sections, and performs various calculating processes. The controlling section 350 includes a storing section (not shown) for storing control programs and various data.

### (Receiver 400)

The receiver 400 may include a receiving section 410, a registering section 420, a displaying section 430, an operating section 440, and a controlling section 450.

The receiving section 410 receives the biological information transmitted from the transmitter 200. The receiving section 410 may further receive the association information transmitted from the setting terminal 300.

The registering section 420 registers the patient identification information and the channel in association with each other by using the association information which is received by the receiving section 410. In the registering section 420, the patient identification information and the channel are stored in association with each other. When the receiver 400 receives the biological information from the transmitter 200, therefore, the receiver 400 can make the received biological information correspondent with the patient identification information.

The displaying section 430 is a display screen such as a liquid crystal display, and displays various information such as the biological information. The displaying section 430 displays the patient biological information of the patient and patient identification information received by the receiving section 410, in correspondence with each other based on the patient identification information and channel which are stored in the registering section 420 in association with each other.

The operating section 440 is configured by an input device such as a mouse or a keyboard, and receives various inputs and the like from the user. Alternatively, the operating section 440 may be configured by operation keys which are realized as software by a touch panel system in the displaying section 430 of the receiver 400.

The controlling section 450 is configured by, for example, a CPU, controls the above-described sections, and performs various calculating processes. The controlling section 450 includes a storing section (not shown) for storing control programs and various data.

Next, the process procedure in the medical system of the first embodiment will be described.

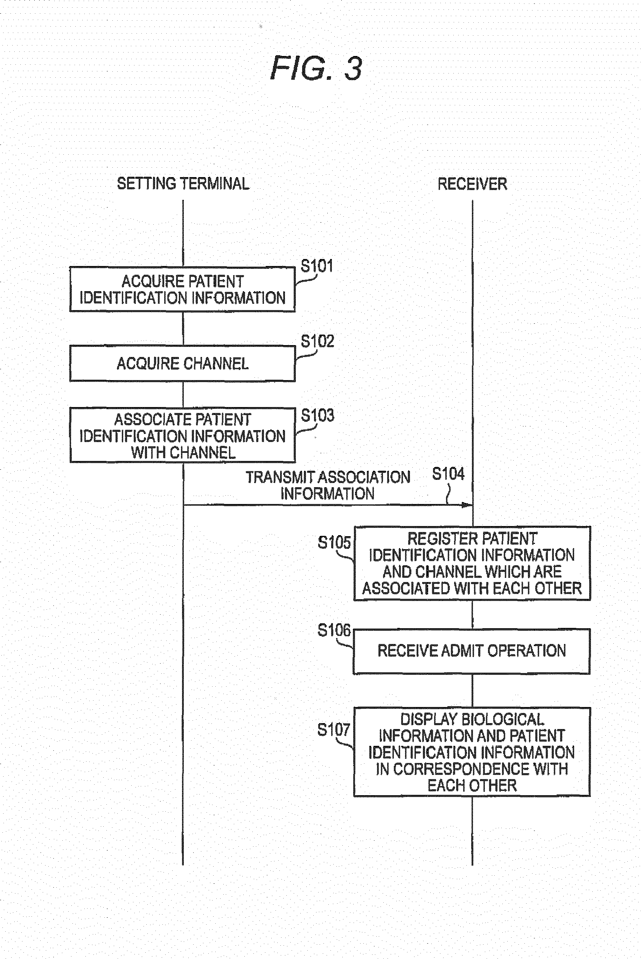

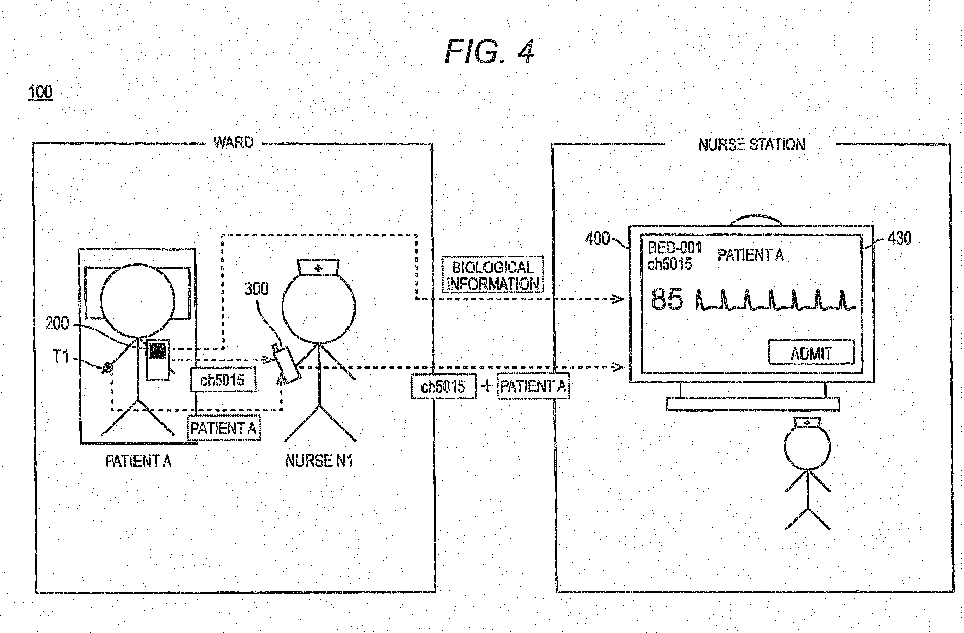

Fig. 3 is a sequence diagram illustrating the process procedure in the medical system of the first embodiment, and Fig. 4 illustrates the manner of processing in the medical system of the first embodiment. The processes of the setting terminal 300 shown in Fig. 3 are stored in the form of programs in the storing section of the setting terminal 300, and are executed when the controlling section 350 controls the sections. The processes of the receiver 400 shown in Fig. 3 are stored in the form of programs in the storing section of the receiver 400, and executed when the controlling section 450 controls the sections.

First, the setting terminal 300 acquires the patient identification information (step S101). In the setting terminal 300, specifically, the patient identification information acquiring section 320 recognizes a patient tag or the like to acquire patient identification information, and the information is stored in the storing section. In the example of Fig. 4, from a patient tag T1 attached to the arm of a patient A, the setting terminal 300 acquires "Patient A" which is the name of the patient, as patient identification information. As described above, the patient tag or the like is recognized by the setting terminal 300, and the patient identification information can be automatically acquired. Therefore, it is not necessary for the medical staff member to manually input the patient identification information. Moreover, the medical staff member can acquire the patient identification information in the vicinity of the patient, and therefore a situation where the medical staff member mistakenly identifies the patient as another patient can be suppressed.

Then, the setting terminal 300 acquires the channel (step S102). Specifically, a nurse N1 moves the setting terminal 300 close to the transmitter 200, and performs an operation such as press of a button of the operating section 340 of the setting terminal 300, whereby the channel of the transmitter 200 is acquired. In the example of Fig. 4, the setting terminal 300 acquires "ch5015" which is the channel used by the transmitter 200 attached to the patient A. In this way, the setting terminal 300 can automatically acquire the channel from the transmitter 200 attached to the patient, and therefore it is not necessary for the medical staff member to manually input the channel. After the medical staff member acquires the patient identification information of the patient, moreover, the channel can be automatically acquired from the transmitter 200 which remains to be attached to the patient. Therefore, the patient identification information and the channel are correctly associated with each other.

Then, the setting terminal 300 stores the patient identification information and the channel in association with each other (step S103). Specifically, the setting terminal 300 stores the patient identification information which is acquired in the process of step S101, and the channel which is acquired in the process of step S102, in association with each other as the association information in the storing section. In the example of Fig. 4, the setting terminal 300 stores the name "Patient A" which is the patient identification information of the patient A, and "ch5015" which is the channel of the transmitter 200 attached to the patient A, in association with each other.

Then, the setting terminal 300 transmits the association information which is stored in the process of step S103, to the receiver 400 (step S104). In the example of Fig. 4, the setting terminal 300 associates the patient identification information "Patient A" with the channel "ch5015", and transmits them as the association information to the receiver 400.

When the receiver 400 receives the association information from the setting terminal 300, the receiver registers the patient identification information and the channel in association with each other by using the received information, in the registering section 420 (step S105). In the example of Fig. 4, the setting terminal 300 registers the patient identification information "Patient A" and the channel "ch5015" in association with each other. When the receiver 400 receives biological information from the transmitter 200, therefore, the receiver can display, for example, the received biological information and the patient identification information "Patient A", in correspondence with each other based on the used channel "ch5015" on the display.

Then, the receiver 400 receives an admit operation which is an operation for starting monitoring of the biological information, from the user (step S106). As shown in Fig. 4, for example, the receiver 400 displays an "Admit " button for receiving the admit operation, on the displaying section 430, and, when the user operates the operating section 440 to press the "Admit" button, receives the admit operation. In the admit operation, in addition to instructions for starting monitoring of the biological information, various settings required for monitoring of the biological information such as settings relating to an alarm which is to be produced when abnormality occurs in the biological information may be received.

Then, the receiver 400 displays the biological information of the patient and the patient identification information in correspondence with each other (step S107). Specifically, based on the patient identification information and channel which are stored in the registering section 420 in association with each other, the receiver 400 displays the biological information and patient identification information which are received from the transmitter 200, in correspondence with each other on the displaying section 430. In the example of Fig. 4, the channel which is used in the reception of the biological information is "ch5015", and therefore the receiver 400 displays the patient identification information "Patient A" which is associated with the channel "ch5015" in the registering section 420, in correspondence with the biological information. Namely, the name "Patient A" which is the patient identification information of the patient A is displayed on the displaying section 430 while being corresponded with the biological information acquired from the patient A. Therefore, the medical staff member who monitors the biological information can easily recognize the biological information displayed on the displaying section 430, as the information of the patient A. Even in the case where a plurality of patients exist, the biological information of each of the patients is displayed in correspondence with the patient identification information of the patients. Therefore, the medical staff member can monitor the sets of biological information of the patients without error.

According to the medical system of the first embodiment, as described above, the setting terminal 300 acquires and associates the patient identification information and the channel with each other, and the receiver 400 registers the patient identification information and channel which are associated with each other in the setting terminal 300. Therefore, the medical staff member can easily and correctly associate the patient identification information with the channel.

Moreover, the setting terminal 300 reads a medium such as the patient tag attached to the patient to automatically recognize and acquire the patient identification information. Therefore, the medical staff member is not need to manually input the patient identification information, and it is possible to suppress an input error.

Although, in the embodiment, the setting terminal 300 acquires the channel after it acquires the patient identification information, the sequence of acquiring these sets of information is not limited to this. The setting terminal 300 may acquire the patient identification information after it acquires the channel.

Although the embodiment has been described on the assumption that the setting terminal 300 and the receiver 400 are configured as independent apparatuses, respectively, the invention is not limited to this. The setting terminal 300 and the receiver 400 may be integrated into one apparatus. According to the configuration, the medical staff member such as a nurse can associate patient identification information with a channel by using the setting terminal 300, and then monitor biological information by using the same apparatus. Therefore, it is possible to rapidly perform a medical practice.

Although the embodiment has been described on the condition that the setting terminal 300 associates patient identification information with a channel, the embodiment is not limited to this. In the case where the receiver 400 receives patient identification information and a channel from the setting terminal 300 at the same timing, the receiver 400 may associate the patient identification information with the channel.

### (Second embodiment)

Next, a medical system of a second embodiment will be described.

In the first embodiment, when the receiver 400 receives an admit operation, monitoring of biological information is started. In an exemplified case where the medical staff member forgets to perform an admit operation through the receiver 400, however, monitoring of biological information is not started. In this case, even when abnormality occurs in a patient, the medical staff member cannot detect the abnormality because monitoring of the biological information is not started. This is problematic. In order to enable an admit operation to be surely performed, also an admit operation may be received after the setting terminal 300 associates patient identification information with a channel. The second embodiment in which the setting terminal 300 receives an admit operation will be described.

The medical system of the second embodiment is configured in a same or a similar manner as that of the first embodiment. Hereinafter, the description of the portions which are identical with those of the first embodiment will be omitted, and the description will be made with emphasis on the portions which are different from those of the first embodiment.

The process procedure in the medical system of the second embodiment will be described.

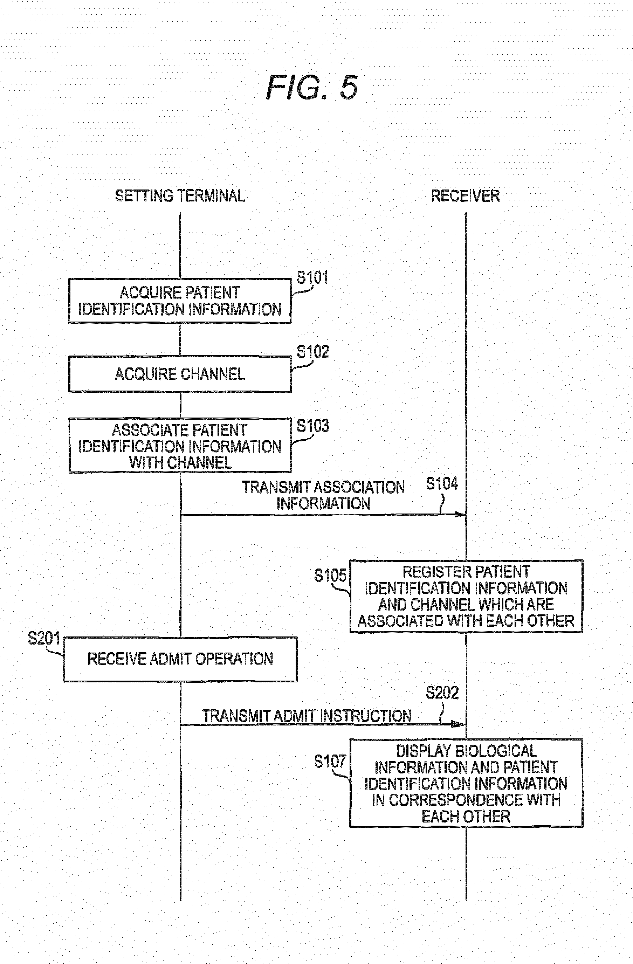

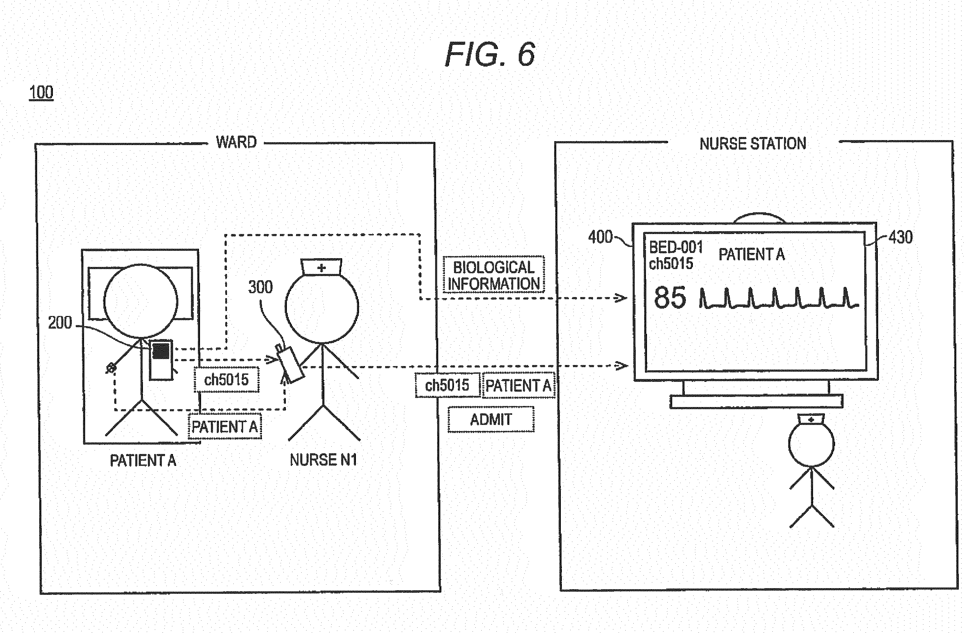

Fig. 5 is a sequence diagram illustrating the process procedure in the medical system of the second embodiment, and Fig. 6 illustrates the manner of processing in the medical system of the second embodiment. The processes of the setting terminal 300 shown in Fig. 5 are stored in the form of programs in the storing section of the setting terminal 300, and are executed when the controlling section 350 controls the sections. The processes of the receiver 400 shown in Fig. 5 are stored in the form of programs in the storing section of the receiver 400, and executed when the controlling section 450 controls the sections. The processes of steps S101 to S105 and S107 of the sequence diagram are identical with those of steps S101 to S105 and S107 in Fig. 3. Hereinafter, the processes of steps S203. and S202 will be mainly described.

As shown in Fig. 5, the setting terminal 300 acquires the patient identification information in the process of step S101, and acquires the channel in the process of step S102. Then, the setting terminal 300 associates the patient identification information with the channel in the process of step S103, and transmits the association information to the receiver 400 in the process of step S104. When the receiver 400 receives the association information from the setting terminal 300, the receiver registers the association information in the process of step S105.

Then, the setting terminal 300 receives an admit operation from the user (step S201). For example, the setting terminal 300 displays the "Admit" button for receiving the admit operation, on the displaying section, and, when the user operates the operating section 340 to press the "Admit" button, receives the admit operation. In the process of receiving the admit operation, various settings required for monitoring of the biological information such as settings relating to an alarm which is to be produced when abnormality occurs in the biological information may be received.

Then, the setting terminal 300 transmits an admit instruction to the receiver 400 as shown in Fig. 6 (step S202). Specifically, the setting terminal 300 transmits instructions for starting the monitoring to the receiver 400 based on the admit operation received in the process of step S201. In the case where various settings required for monitoring of the biological information are received in the process of step S201, the setting terminal 300 transmits also information relating to the settings to the receiver 400

When the receiver 400 receives the admit instruction from the setting terminal 300, the receiver displays the biological information of the patient and the patient identification information in correspondence with each other in the process of step S107.

According to the medical system of the second embodiment, as described above, the setting terminal 300 receives the admit operation for starting monitoring of the biological information of the patient. Therefore, the admit operation can be executed together with the process of associating the patient identification information with the channel, and therefore a situation where the medical staff member forgets to perform an admit operation can be suppressed.

The process of receiving the admit operation in step S201 in Fig. 5 may be performed together with the processes of steps S101 to S103. In this case, when the setting terminal 300 transmits the association information in the process of step S104, the setting terminal can transmit also the admit instruction. In the admit operation, therefore, the labor of the medical staff member and the process time of the system can be reduced.

In the process of step S201 in Fig. 5, before the "Admit" button is displayed on the displaying section, the setting terminal 300 may inquire of the receiver 400 whether the admit operation is completed or not. Hereinafter, an admit completion inquiring process which is executed by the setting terminal 300 will be described.

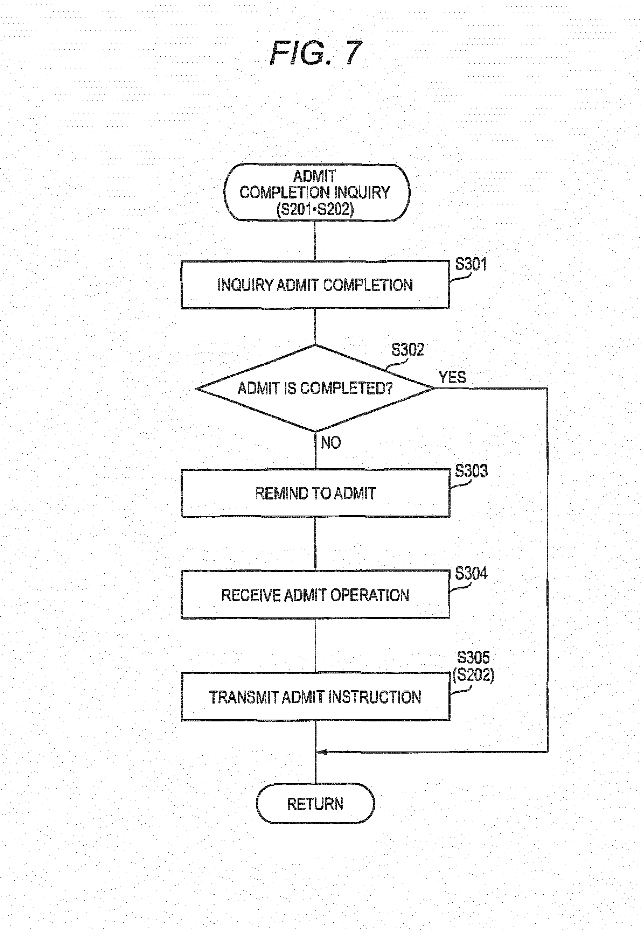

Fig. 7 is a flowchart illustrating the procedure of an admit completion inquiring process which is performed in the admit instruction receiving process shown in steps S201 and S202 in Fig. 5. The processes shown in Fig. 7 are stored in the form of programs in the storing section of the setting terminal 300, and are executed when the controlling section 350 controls the sections.

As shown in Fig. 7, the setting terminal 300 inquires of the receiver 400 whether the admit operation for the target patient is completed or not (step S301). Based on a result of the inquiry in step S301, the setting terminal 300 determines whether the admit operation for the patient who is targeted is completed in the receiver 400 or not (step S302).

If the admit operation is completed (step S302: YES), the setting terminal 300 omits the admit operation receiving process in which the display of the "Admit" button and the like are performed, and terminates the admit completion inquiring process. The case where, for example, the receiver 400 receives the admit operation as in step S106 in the first embodiment corresponds to the above-described case. In this case, the admit operation has been already completed, and therefore the receiver 400 displays the biological information of the patient and the patient identification information in correspondence with each other in the process of step S107, without waiting for the transmission of the admit instruction shown in step S202 in Fig. 5.

If the admit operation is not completed (step S302: NO), by contrast, the setting terminal 300 reminds the user to perform the admit operation (step S303). Specifically, the setting terminal 300 displays a message for prompting the user to perform the admit operation, on the displaying section, or sounds an alarm, thereby reminding the user to perform the operation.

Then, the setting terminal 300 receives the admit operation from the user (step S304. The process of receiving the admit operation is similar to that of step S201 in Fig. 5. When the setting terminal 300 receives the admit operation, the terminal transmits the admit instruction to the receiver 400 (step S305). The process of transmitting the admit instruction is similar to that of step S201 in Fig. 5. At every elapse of a predetermined time period, the setting terminal 300 may perform the admit completion inquiring process, and remind to execute the admit operation until the admit operation is completed. According to the configuration, a situation where the medical staff member forgets to perform an admit operation can be suppressed more surely.

According to the invention, the setting terminal acquires and associates patient identification information and a channel with each other, and the receiver registers the patient identification information and channel which are associated with each other in the setting terminal, thereby enabling a medical staff member to easily and correctly associate the patient identification information with the channel.

## Claims

1. A medical system comprising:
a transmitter which is configured to acquire biological information of a patient, and which is configured to transmit the biological information through an inherent channel;
a setting terminal which is configured to recognize identification information for identifying a patient, which is configured to acquire the channel from the transmitter, and which is configured to transmit association information in which the identification information is associated with the channel; and
a receiver which, by using the association information transmitted from the setting terminal, is configured to register the identification information and the channel in association with each other.

2. The medical system according to claim 1, wherein
the setting terminal is configured to receive an admit operation for starting monitoring of the biological information of the patient, and is configured to instruct the receiver to start the monitoring, based on the received admit operation.

3. The medical system according to claim 1 or 2, wherein
the setting terminal is configured to inquire whether an admit operation for starting monitoring of the biological information of the patient is completed in the receiver or not, and, when the admit operation is not completed, is configured to perform notification for prompting completion of the admit operation.

4. The medical system according to any one of claims 1 to 3, wherein
the setting terminal is configured to read a medium attached to the patient, to recognize the identification information.
